# EUROPEAN PATENT SPECIFICATION

(11) **EP 4 164 508 B1**
(45) Date of publication and mention of the grant of the patent: **03.04.2024**
(21) Application number: 22721883.1
(22) Date of filing: 27.04.2022
(51) Int. Cl.: A61B 17/064, A61B 17/072, A61B 18/14, A61B 17/00, A61B 17/29, A61B 90/00, A61B 18/00

(54) **SHAFT SYSTEM FOR SURGICAL INSTRUMENT**
SCHAFTSYSTEM FÜR EIN CHIRURGISCHES INSTRUMENT
SYSTÈME D'ARBRE POUR INSTRUMENT CHIRURGICAL

(30) Priority: 30.04.2021 US 202117246141
(43) Date of publication of application: 19.04.2023
(73) Proprietor: Cilag GmbH International, 6300 Zug (CH)
(72) Inventor: SHELTON, IV, Frederick E., Cincinnati, Ohio 45242 (US); ADAMS, Shane R., Cincinnati, Ohio 45242 (US); ARONHALT, Taylor W., Cincinnati, Ohio 45242 (US)
(74) Representative: Carpmaels & Ransford LLP
(86) International application number: PCT/IB2022/053896
(87) International publication number: WO 2022/229866

(56) References cited:
- EP-A2- 1 006 885
- EP-A2- 3 120 781
- EP-B1- 1 006 885
- US-A1- 2005 261 692
- US-A1- 2009 018 567

## Description

### BACKGROUND

The present invention relates to surgical instruments and, in various arrangements, to surgical stapling and cutting instruments and staple cartridges for use therewith that are designed to staple and cut tissue.

WO98/17190 describes a catheter that includes a handle, a visual orientation indicator, a stem with depth indicators, a heater portion, and a tip. The stem is divided into a proximal section, a transition section, and a distal-heater section. The proximal section includes an outer polyimide sheath, a stainless-steel tube, a stainless-steel core, thermocouple conductors, and electrical conductors. The proximal section of the catheter resists both bending and buckling as the catheter is inserted into a lumen. In the transition section of the catheter, the stainless-steel tube is replaced with a relatively flexible polyimide tube. This is housed within outer polyimide sheath. Both of these tubes are flexible. Nevertheless, the transition section has a preferred bending axis. Which is orthogonal to the longitudinal axis of a stainless-steel cross-member. This cross-member provides resistance to deflection along one axis while allowing deflection along another axis. At the distal heater section of the catheter, the outer polyimide sheath wraps around a resistive heating coil.

### SUMMARY

The present invention provides a surgical instrument assembly according to claim 1. The surgical instrument assembly comprises a shaft, an articulation region, and an end effector attached to the shaft by way of the articulation region. The end effector is configured to be articulated relative to the shaft. The shaft comprises an outer housing, an inner spine positioned with the outer housing, and a core member positioned within the inner spine. The core member comprises a distal end attached to a component the end effector, a proximal end attached to a proximal housing of the surgical instrument assembly. The core member comprises a material different than a material of the inner spine. The core member defines a maximum system stretch of the inner spine. The core member comprises a proximal core member and a distal core member. The proximal core member is attached to the proximal housing and the distal core member is attached to the component of the end effector.

### BRIEF DESCRIPTION OF THE DRAWINGS

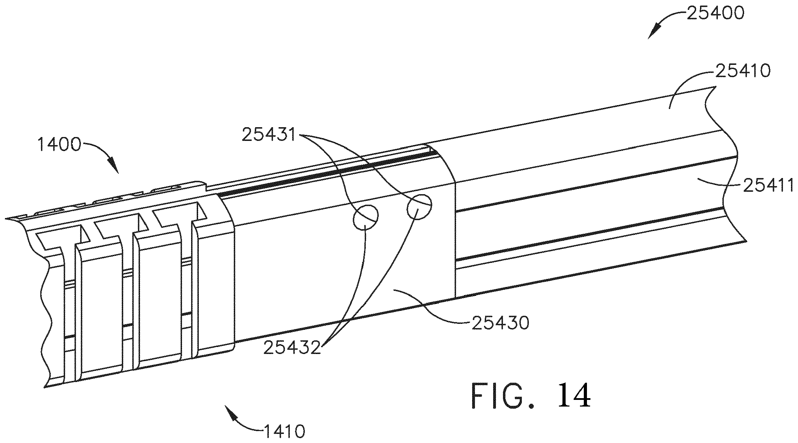

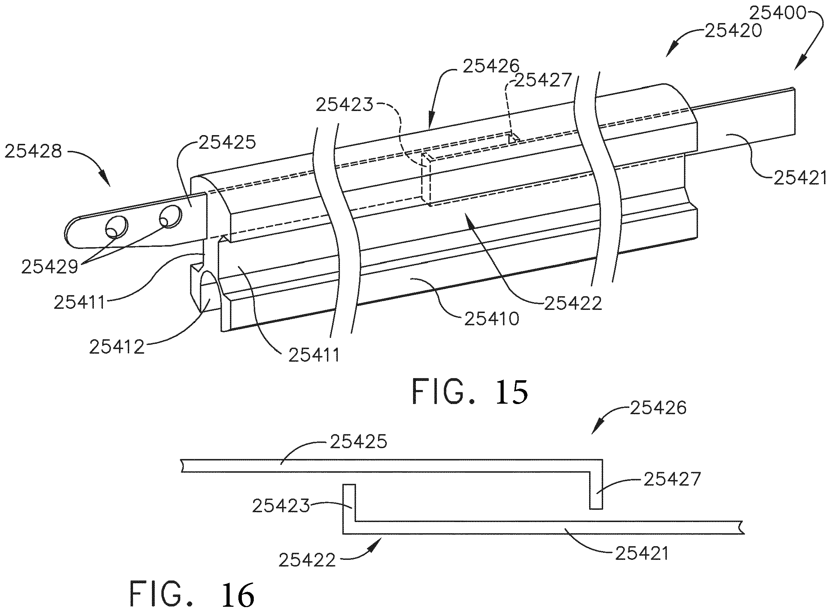

Various features of the devices described herein, together with advantages thereof, may be understood in accordance with the following description taken in conjunction with the accompanying drawings. The present invention is illustrated in FIGS. 14 - 16 of the drawings, and the remaining figures are present to provide context for the invention, as follows:
FIG. 1 is a perspective view of surgical instrument;
FIG. 2 is a perspective view of a shaft of the surgical instrument of FIG. 1;

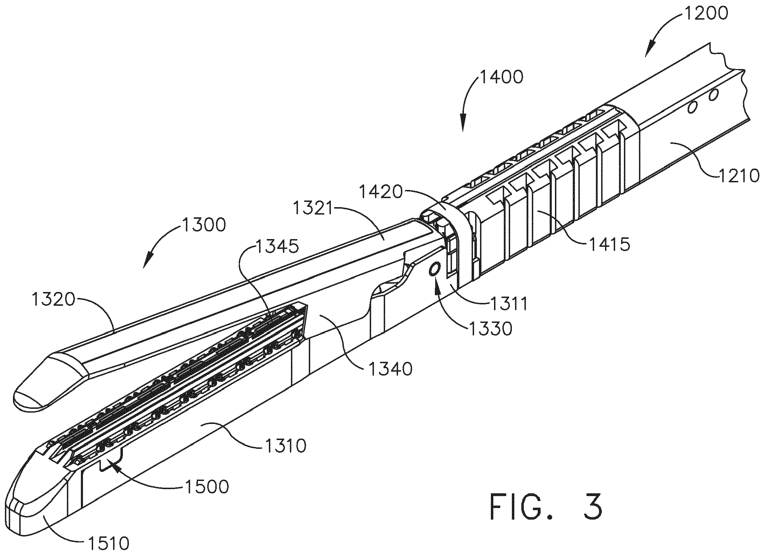

FIG. 3 is a perspective view of an end effector of the surgical instrument of FIG. 1;
FIG. 4 is a partial exploded view of the shaft of FIG. 2;
FIG. 5 is a partial exploded view of the end effector of FIG. 3;
FIG. 6 is an exploded view of the end effector of FIG. 3;
FIG. 7 is an elevational view of the end effector of FIG. 3 illustrating the end effector in an open configuration;
FIG. 8 is an elevational view of the end effector of FIG. 3 illustrating the end effector in a closed configuration;

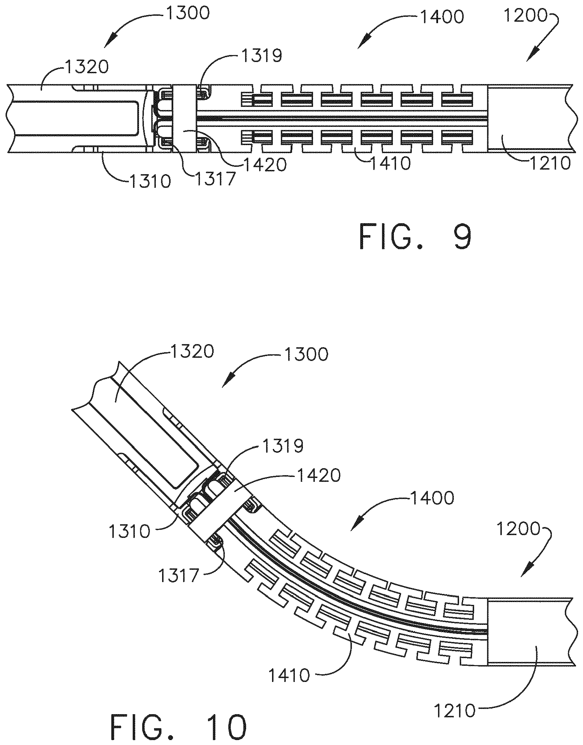

FIG. 9 is a plan view of an articulation joint of the surgical instrument of FIG. 1 illustrating the end effector in an unarticulated position;
FIG. 10 is a plan view of an articulation joint of the surgical instrument of FIG. 1 illustrating the end effector in an articulated position;
FIG. 11 is a cross-sectional view of the end effector of FIG. 3 illustrated in a closed, unfired configuration;
FIG. 12 is a cross-sectional view of the end effector of FIG. 3 illustrated in a closed, fired configuration;
FIG. 12A is a cross-sectional view of the end effector of FIG. 3 illustrated in a open configuration;
FIG. 13 is a perspective view of a handle of the surgical instrument of FIG. 1 illustrated with some components removed;
FIG. 14 is a perspective view of a surgical instrument shaft assembly comprising a spine, an articulation joint, and a core insert positioned within the spine;
FIG. 15 is a perspective view of the spine and core inset of FIG. 14, wherein the core insert comprises a proximal core member and a distal core member positioned within the spine;
FIG. 16 is a partial elevational view of the proximal core member and the distal core member of the surgical instrument assembly of FIG. 14;

Corresponding reference characters indicate corresponding parts throughout the several views. The exemplifications set out herein are not to be construed as limiting the scope of the invention in any manner.

### DETAILED DESCRIPTION

Numerous specific details are set forth to provide a thorough understanding of the overall structure, function, manufacture, and use of the devices as described in the specification and illustrated in the accompanying drawings. Well-known operations, components, and elements have not been described in detail so as not to obscure the devices described in the specification. The reader will understand that the devices described and illustrated herein are non-limiting examples, and thus it can be appreciated that the specific structural and functional details disclosed herein may be representative and illustrative. Variations and changes thereto may be made without departing from the scope of the claims.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a surgical system, device, or apparatus that "comprises," "has," "includes" or "contains" one or more elements possesses those one or more elements, but is not limited to possessing only those one or more elements. Likewise, an element of a system, device, or apparatus that "comprises," "has," "includes" or "contains" one or more features possesses those one or more features, but is not limited to possessing only those one or more features.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices are provided for performing laparoscopic and minimally invasive surgical procedures. However, the reader will readily appreciate that the various devices disclosed herein can be used in numerous surgical procedures and applications including, for example, in connection with open surgical procedures. As the present Detailed Description proceeds, the reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongate shaft of a surgical instrument can be advanced.

A surgical stapling system can comprise a shaft and an end effector extending from the shaft. The end effector comprises a first jaw and a second jaw. The first jaw comprises a staple cartridge. The staple cartridge is insertable into and removable from the first jaw; however, other possibilities are envisioned in which a staple cartridge is not removable from, or at least readily replaceable from, the first jaw. The second jaw comprises an anvil configured to deform staples ejected from the staple cartridge. The second jaw is pivotable relative to the first jaw about a closure axis; however, other possibilities are envisioned in which the first jaw is pivotable relative to the second jaw. The surgical stapling system further comprises an articulation joint configured to permit the end effector to be rotated, or articulated, relative to the shaft. The end effector is rotatable about an articulation axis extending through the articulation joint. Other possibilities are envisioned which do not include an articulation joint.

The staple cartridge comprises a cartridge body. The cartridge body includes a proximal end, a distal end, and a deck extending between the proximal end and the distal end. In use, the staple cartridge is positioned on a first side of the tissue to be stapled and the anvil is positioned on a second side of the tissue. The anvil is moved toward the staple cartridge to compress and clamp the tissue against the deck. Thereafter, staples removably stored in the cartridge body can be deployed into the tissue. The cartridge body includes staple cavities defined therein wherein staples are removably stored in the staple cavities. The staple cavities are arranged in six longitudinal rows. Three rows of staple cavities are positioned on a first side of a longitudinal slot and three rows of staple cavities are positioned on a second side of the longitudinal slot. Other arrangements of staple cavities and staples may be possible.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions by a sled. The sled is movable between a proximal position adjacent the proximal end and a distal position adjacent the distal end. The sled comprises a plurality of ramped surfaces configured to slide under the drivers and lift the drivers, and the staples supported thereon, toward the anvil.

Further to the above, the sled is moved distally by a firing member. The firing member is configured to contact the sled and push the sled toward the distal end. The longitudinal slot defined in the cartridge body is configured to receive the firing member. The anvil also includes a slot configured to receive the firing member. The firing member further comprises a first cam which engages the first jaw and a second cam which engages the second jaw. As the firing member is advanced distally, the first cam and the second cam can control the distance, or tissue gap, between the deck of the staple cartridge and the anvil. The firing member also comprises a knife configured to incise the tissue captured intermediate the staple cartridge and the anvil. It is desirable for the knife to be positioned at least partially proximal to the ramped surfaces such that the staples are ejected ahead of the knife.

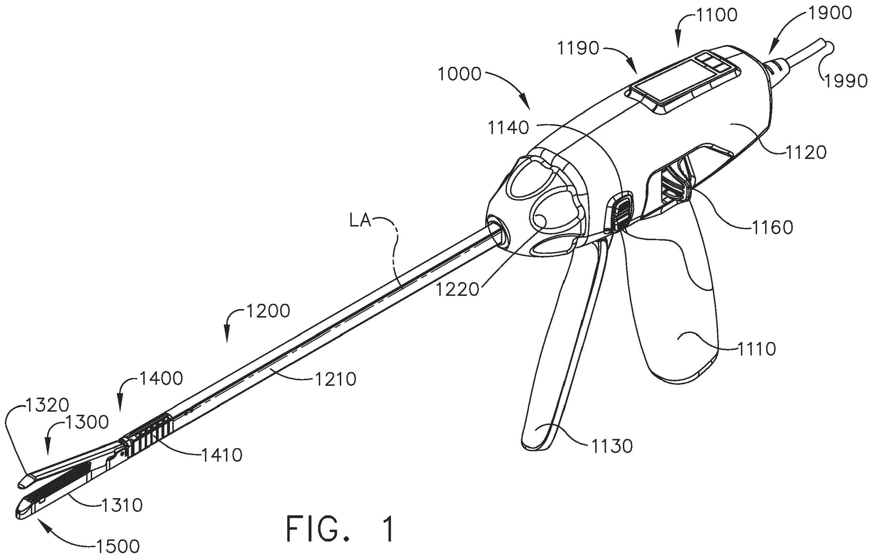

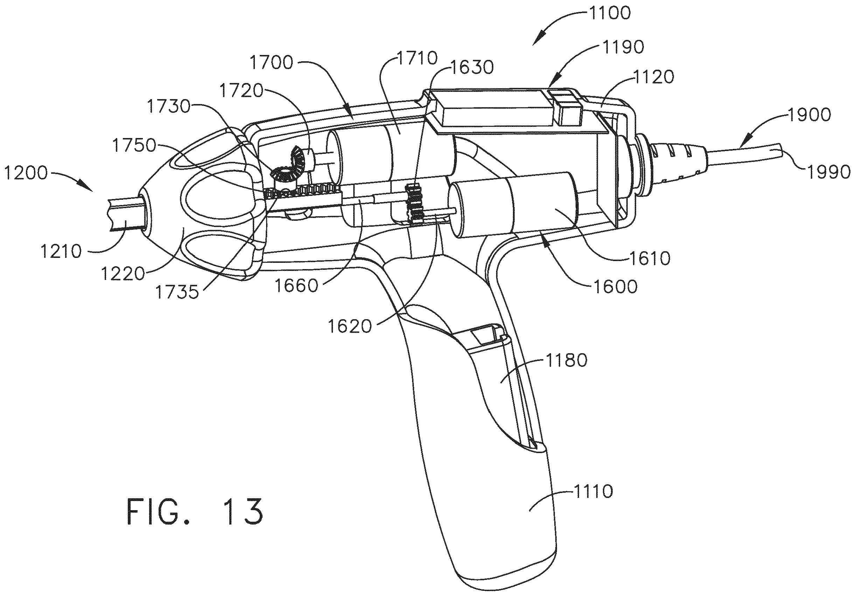

A surgical instrument 1000 is illustrated in FIG. 1. As discussed in greater detail below, the surgical instrument 1000 is configured to clamp, incise, and seal patient tissue. The surgical instrument 1000 comprises an end effector 1300, an articulation joint 1400, and an articulation drive system 1700 (FIG. 13) configured to articulate the end effector 1300 about the articulation joint 1400. The end effector 1300 comprises a first jaw 1310, a second jaw 1320 movable between an open position and a closed position, and a drive system 1600 (FIG. 13) operable to close the second jaw 1320 during a closure stroke. After the end effector 1300 is closed, the drive system 1600 is operable once again to incise and staple the patient tissue captured between the first jaw 1310 and the second jaw 1320 during a firing stroke. Additionally, the surgical instrument 1000 comprises an energy delivery system 1900 which is also operable to seal the incised tissue.

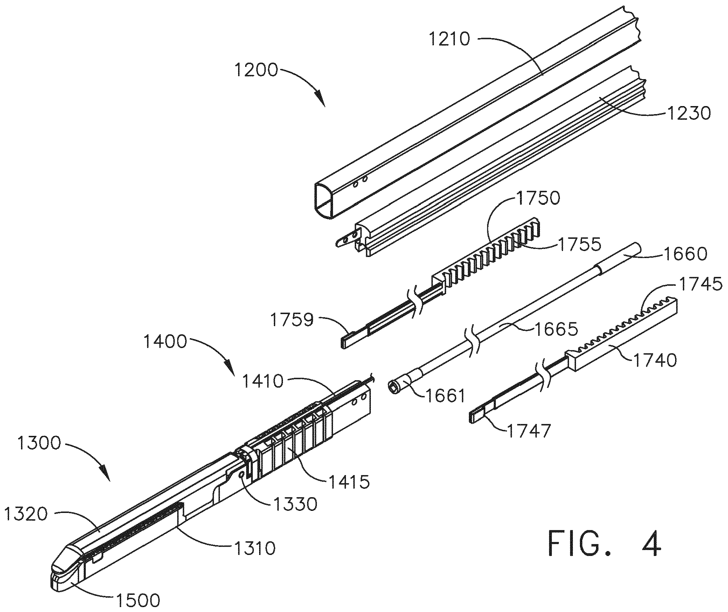

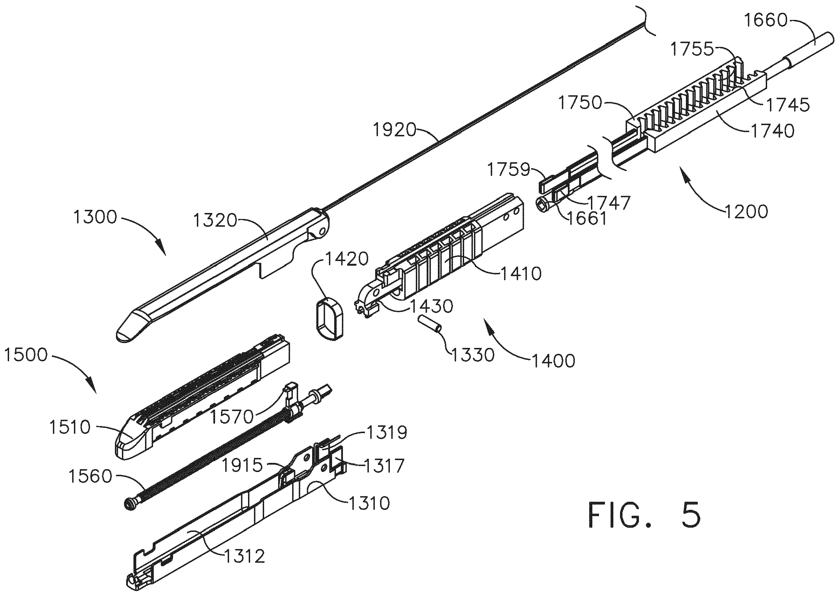

The surgical instrument 1000 further comprises a handle 1100 and a shaft 1200 extending from the handle 1100. The handle 1100 comprises a grip 1110 extending downwardly from a handle body 1120. As discussed in greater detail below, the handle 1100 further comprises a closure actuator 1130 operable to close the end effector 1300 and an articulation actuator 1140 operable to articulate the end effector 1300 relative to the shaft 1200. The shaft 1200 comprises an outer housing 1210 and an inner frame, or spine, 1230 (FIG. 4) which are rotatably mounted to the handle body 1120 about a rotation joint 1220. Referring to FIG. 5, the articulation joint 1400 comprises a flexible outer housing 1410 affixed to the outer housing 1210 and a flexible articulation frame 1430 connected to the shaft frame 1230 (FIG. 4). The first jaw 1310 comprises a proximal end 1311 mounted to the flexible articulation frame 1430 via a pin forming a rotation joint 1330 between the first jaw 1310 and the second jaw 1320. The distal end of the flexible articulation housing 1410 is also affixed to the first jaw 1310 via a clamp ring 1420 such that the end effector 1300 is affixed to the distal end of the articulation joint 1400.

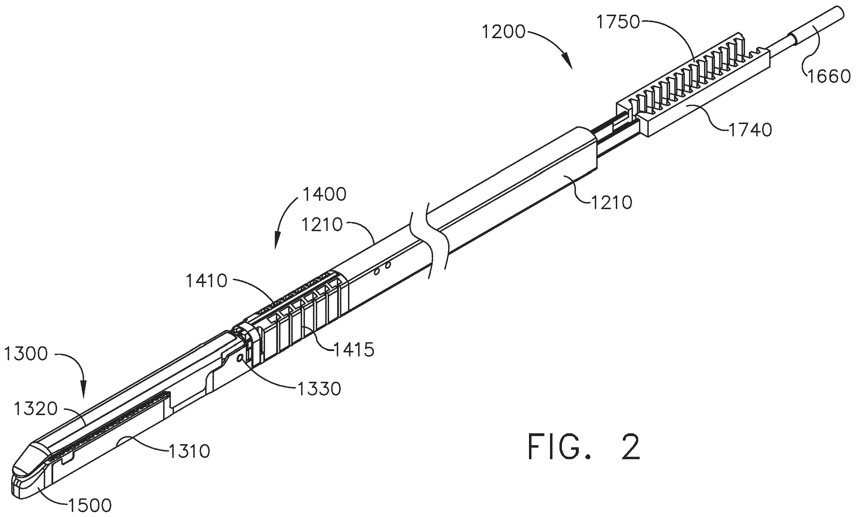

Referring primarily to FIGS. 1 and 13, the articulation drive system 1700 comprises an electric motor 1710 in communication with a control system of the surgical instrument 1000. The control system is configured to supply power to the electric motor 1710 from a battery 1180 in response to an actuation of the articulation actuator 1140. The articulation actuator 1140 comprises a switch that is actuatable in a first direction to operate the electric motor 1710 in a first direction and actuatable in a second direction to operate the electric motor 1710 in a second, or opposite, direction. The articulation drive system 1700 further comprises a transfer gear 1730 rotatably supported within the handle 1100 that is operably engaged with a gear output 1720 of the electric motor 1710. Referring primarily to FIGS. 2 and 4, the articulation drive system 1700 also comprises a first articulation actuator 1740 and a second articulation actuator 1750 operably engaged with the transfer gear 1730. More specifically, the transfer gear 1730 comprises a pinion gear portion 1735 intermeshed with a first drive rack 1745 defined on the proximal end of the first articulation actuator 1740 and a second drive rack 1755 defined on the proximal end of the second articulation actuator 1750. Owing to the positioning of the first drive rack 1745 and the second drive rack 1755 on opposite sides of the transfer gear 1730, the first articulation actuator 1740 and the second articulation actuator 1750 are driven proximally and distally in opposition to, or antagonistically with respect to, one another when the transfer gear 1730 is rotated. For instance, the first articulation actuator 1740 is driven distally and the second articulation actuator 1750 is driven proximally to articulate the end effector 1300 in a first direction when the electric motor 1710 is operated in its first direction. Correspondingly, the first articulation actuator 1740 is driven proximally and the second articulation actuator 1750 is driven distally to articulate the end effector 1300 in a second, or opposite, direction when the electric motor 1710 is operated in its second direction.

Referring to FIGS. 4-6, the first jaw 1310 comprises a first articulation drive post 1317 extending upwardly on a first lateral side of the first jaw 1310 and a second articulation drive post 1319 extending upwardly on a second, or opposite, lateral side of the first jaw 1310. The distal end of the first articulation actuator 1740 comprises a first drive mount 1747 engaged with the first articulation drive post 1317 and the distal end of the second articulation actuator 1750 comprises a second drive mount 1759 engaged with the second articulation drive post 1319 such that the proximal and distal movement of the articulation actuators 1740 and 1750 rotate the end effector 1300 about the articulation joint 1400. When the end effector 1300 is articulated, the flexible outer housing 1410 and the flexible articulation frame 1430 of the articulation joint 1400 resiliently deflect to accommodate the rotation of the end effector 1300. In various instances, the articulated position of the end effector 1300 is held in place due to friction within the articulation drive 1700. In some cases, the articulation drive 1700 comprises an articulation lock configured to releasably hold the end effector 1300 in position.

As discussed above, the surgical instrument 1000 comprises a drive system 1600 operable to close the end effector 1300 and then operable once again to staple and incise the patient tissue captured between the first jaw 1310 and the second jaw 1320 of the end effector 1300. Referring again to FIG. 13, the drive system 1600 comprises an electric motor 1610 in communication with the control system of the surgical instrument 1000. The control system is configured to supply power to the electric motor 1610 from the battery 1180 in response to an actuation of the closure actuator 1130. The closure actuator 1130 comprises a switch that is actuatable in a first direction to operate the electric motor 1610 in a first direction to close the end effector 1300 and actuatable in a second direction to operate the electric motor 1610 in a second, or opposite, direction to open the end effector 1300. The closure drive system 1600 further comprises a transfer gear 1630 rotatably supported within the handle 1100 that is operably engaged with a gear output 1620 of the electric motor 1610. The transfer gear 1630 is fixedly mounted to a rotatable drive shaft 1660 such that the drive shaft 1660 rotates with the transfer gear 1630. The rotatable drive shaft 1660 extends through the shaft 1200 and comprises a flexible portion 1665 that extends through the articulation joint 1400 to accommodate the articulation of the end effector 1300. The rotatable drive shaft 1660 further comprises a distal coupling 1661 that extends into a proximal end 1311 of the first jaw 1310. In some cases, the distal coupling 1661 comprises a hex-shaped aperture, for example, but could comprise any suitable configuration.

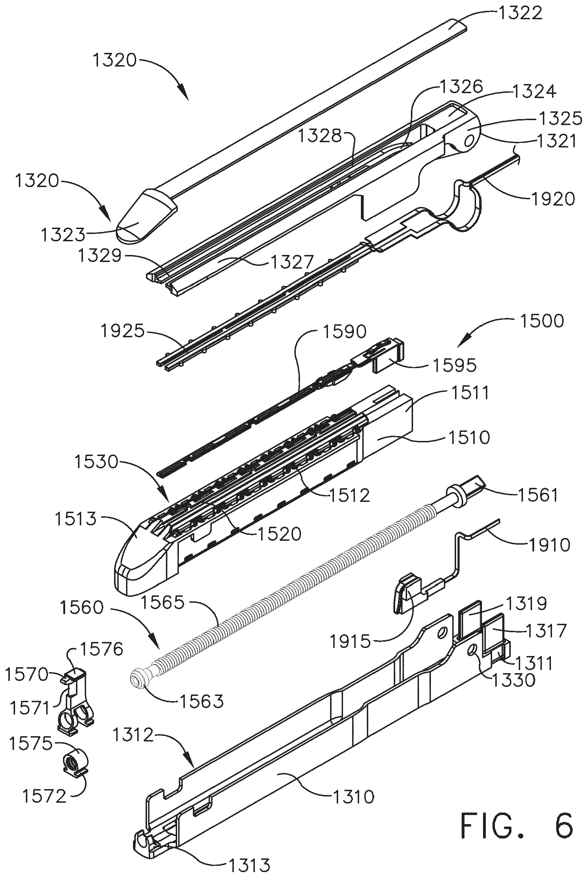

Referring primarily to FIG. 6, the first jaw 1310 further comprises a channel 1312 extending between the proximal end 1311 and a distal end 1313. The channel 1312 comprises two sidewalls extending upwardly from a bottom wall and is configured to receive a staple cartridge, such as a staple cartridge 1500, for example, between the sidewalls. The staple cartridge 1500 comprises a cartridge body 1510 including a proximal end 1511, a distal nose 1513, and a tissue-supporting deck 1512 extending between the proximal end 1511 and the distal nose 1513. The cartridge body 1510 further comprises a longitudinal slot 1520 defined therein extending from the proximal end 1511 toward the distal nose 1513. The cartridge body 1510 also comprises longitudinal rows of staple cavities 1530 extending between the proximal end 1511 and the distal nose 1513. More specifically, the cartridge body 1510 comprises a single longitudinal row of staple cavities 1530 positioned on a first lateral side of the longitudinal slot 1520 and a single longitudinal row of staple cavities 1530 positioned on a second, or opposite, lateral side of the longitudinal slot 1520. That said, a staple cartridge can comprise any suitable number of longitudinal rows of staple cavities 1530. The staple cartridge 1500 further comprises a staple removably stored in each staple cavity 1530 which is ejected from the staple cartridge 1500 during a staple firing stroke, as discussed in greater detail further below.

Further to the above, referring again to FIG. 6, the staple cartridge 1500 further comprises a drive screw 1560 rotatably supported in the cartridge body. More specifically, the drive screw 1560 comprises a proximal end 1561 rotatably supported by a proximal bearing in the proximal end 1511 of the cartridge body 1510 and a distal end 1563 rotatably supported by a distal bearing in the distal end 1513 of the cartridge body 1510. The proximal end 1561 of the drive screw 1560 comprises a hex coupling extending proximally with respect to the proximal end 1511 of the cartridge end 1510. When the staple cartridge 1500 is seated in the first jaw 1310, the proximal end 1511 of the cartridge body 1510 is slid into the proximal end 1311 of the first jaw 1310 such that the hex coupling of the proximal drive screw end 1561 is inserted into and is operably engaged with the distal drive end 1661 of the rotatable drive shaft 1660. Once the drive screw 1560 is coupled to the rotatable drive shaft 1660 the distal nose 1513 of the staple cartridge 1500 is seated in the distal end 1313 of the first jaw 1310. That said, the staple cartridge 1500 can be seated in the first jaw 1310 in any suitable manner. In various instances, the staple cartridge 1500 comprises one or more snap-fit and/or press-fit features which releasably engage the first jaw 1310 to releasably secure the staple cartridge 1500 within the first jaw 1310. To remove the staple cartridge 1500 from the first jaw 1310, an upward, or lifting, force can be applied to the distal nose 1513 of the staple cartridge 1500 to release the staple cartridge 1500 from the first jaw 1310.

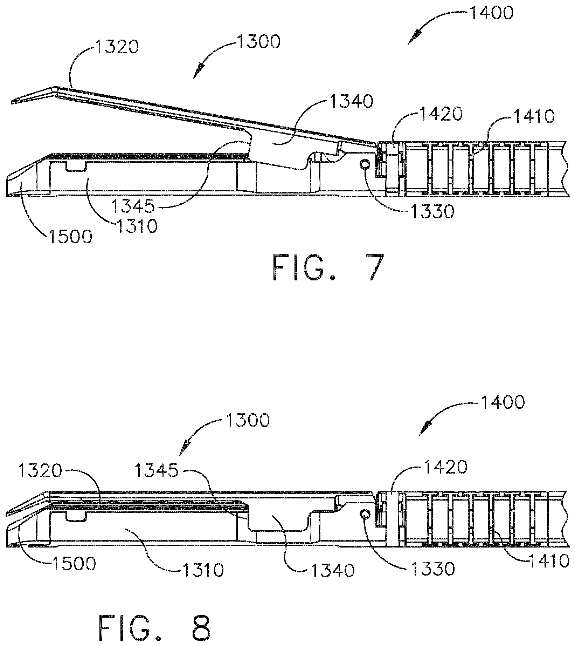

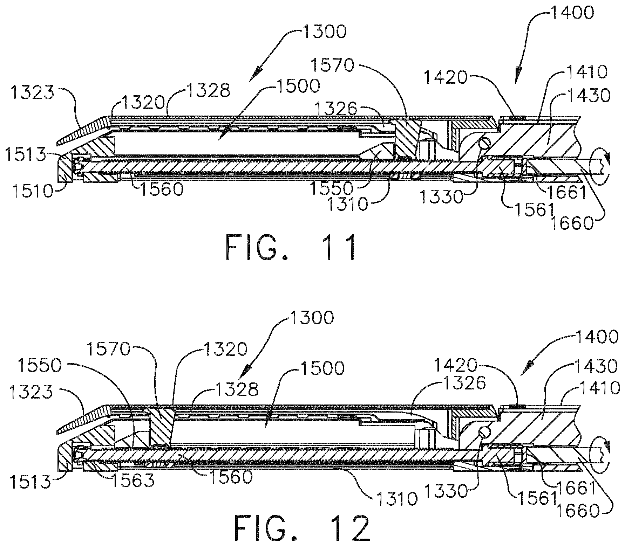

Referring again to FIG. 6, the drive screw 1560 further comprises a threaded portion 1565 extending between the proximal end 1561 and the distal end 1563 and the staple cartridge 1500 further comprises a firing member 1570 threadably engaged with the threaded portion 1565. More specifically, the firing member 1570 comprises a threaded nut insert 1575 threadably engaged with the threaded portion 1565 which is constrained, or at least substantially constrained, from rotating such that the firing member 1570 translates within the staple cartridge 1500 when the drive screw 1560 is rotated. When the drive screw 1560 is rotated in a first direction, the firing member 1570 translates from a proximal unactuated position to a distal actuated position during a closure stroke to move the second jaw 1320 from its open, or unclamped, position (FIG. 7) to its distal, or clamped, position (FIGS. 8 and 11). More specifically, the firing member 1570 comprises a first cam 1572 that engages the first jaw 1310 and a second cam 1576 that engages the second jaw 1320 during the closure stroke which co-operatively position the second jaw 1320 relative to the first jaw 1310. At the outset of the closure stroke, the second cam 1576 is not engaged with the second jaw 1320; however, the second cam 1576 comes into contact with a ramp 1326 (FIG. 6) during the closure stroke to rotate the second jaw 1320 toward the first jaw 1310.

Once the firing member 1570 has reached the end of its closure stroke (FIGS. 8 and 11), the controller of the surgical instrument 1000 stops the drive motor 1610 (FIG. 13). At such point, referring to FIG. 11, the firing member 1570 has not incised the patient tissue captured between the first jaw 1310 and the second jaw 1320 and/or stapled the patient tissue. If the clinician is unsatisfied with the positioning of the jaws 1310 and 1320 on the patient tissue, the clinician can release the closure actuator 1130 (FIG. 1) to operate the drive motor 1610 in its second, or opposite, direction and translate the firing member 1570 proximally out of engagement with the second jaw 1320. In at least one instance, the handle 1100 comprises a closure lock which releasably holds the closure actuator 1130 in its closed position and the clinician must deactivate the closure lock to reopen the closure actuator 1130. Referring to FIG. 1, the handle 1100 further comprises a closure lock release 1160 that, when actuated, unlocks the closure actuator 1130. Once the end effector 1300 is open, the clinician can re-position the end effector 1300 relative to the patient tissue and, once satisfied with the re-positioning of the end effector 1300 relative to the patient tissue, close the closure actuator 1130 once again to re-operate the drive motor 1610 in its first direction to re-close the second jaw 1320. At such point, the drive system 1600 can be operated to perform the staple firing stroke, as discussed further below.

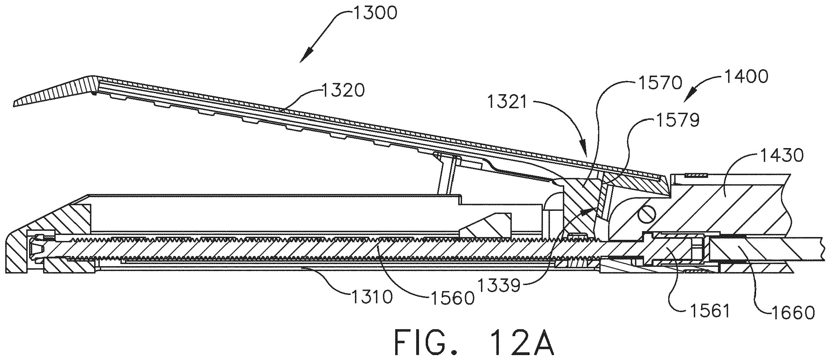

Further to the above, as illustrated in FIG. 11, the firing member 1570 moves into contact with, or in close proximity to, a sled 1550 contained in the cartridge body 1510 at the end of the closure stroke. The surgical instrument 1000 further comprises a firing actuator in communication with the control system of the surgical instrument 1000 which, when actuated, causes the control system to operate the drive motor 1610 in its first direction to advance the firing member 1570 distally from its distal clamped position (FIGS. 8 and 11) and push the sled 1550 through the staple firing stroke, as illustrated in FIG. 12. In some cases, the staple cartridge 1500 comprises staple drivers which support and drive the staples out of the cartridge body 1510 when the staple drivers are contacted by the sled 1550 during the staple firing stroke. In other cases, as discussed in greater detail below, the staples comprise drivers integrally-formed thereon which are directly contacted by the sled 1550 during the staple firing stroke. In either event, the sled 1550 progressively ejects, or fires, the staples out of the cartridge body 1510 as the sled 1550 is moved from its distal clamped position (FIG. 11) to its distal fired position (FIG. 12) by the firing member 1570. Moreover, referring to FIG. 6, the firing member 1570 comprises a tissue cutting edge 1571 that moves through the longitudinal slot 1520 during the staple firing stroke to incise the tissue captured between the deck 1512 of the staple cartridge 1500 and the second jaw 1320.

Further to the above, the second jaw 1320 comprises a frame 1325 including a proximal end 1321 rotatably connected to the first jaw 1310, a longitudinal recess 1324, and a longitudinal slot 1329 configured to receive the firing member 1570 during the staple firing stroke. The frame 1325 further comprises a first longitudinal cam shoulder 1327 defined on a first side of the longitudinal slot 1329 and a second longitudinal cam shoulder 1328 defined on a second side of the longitudinal slot 1329. During the staple firing stroke, the second cam 1576 of the firing member 1570 slides along the first longitudinal cam shoulder 1327 and the second longitudinal cam shoulder 1328 which co-operates with the first cam 1572 to hold the second jaw 1320 in position relative to the first jaw 1310. The frame 1325 also comprises longitudinal rows of staple forming cavities defined therein which are registered with the staple cavities 1530 defined in the staple cartridge 1500 when the second jaw 1320 is in its closed position. The second jaw 1320 further comprises a cover, or cap, 1322 positioned in the longitudinal recess 1324 which is welded to the frame 1325 to enclose the longitudinal slot 1329 and extend over the second cam 1576. The cover 1322 comprises a distal end, or nose, 1323 which is angled toward the distal nose 1513 of the staple cartridge 1500.

In various instances, the clinician can depress and hold the firing actuator until the staple firing stroke is completed. When the firing member 1570 reaches the end of the staple firing stroke, in such instances, the control system can automatically switch the operation of the drive motor 1610 from its first direction to its second direction to retract the firing member 1570 proximally back into its distal clamped position (FIGS. 8 and 11). In such instances, the end effector 1300 remains in its closed, or clamped, configuration until the closure lock release 1160 (FIG. 1) is actuated by the clinician to re-open the closure actuator 1130 and the end effector 1300. In certain instances, the clinician can release the firing actuator prior to the end of the staple firing stroke to stop the drive motor 1610. In such instances, the clinician can re-actuate the firing actuator to complete the staple firing stroke or, alternatively, actuate a retraction actuator in communication with the control system to operate the drive motor 1610 in its second direction to retract the firing member 1570 back into its distal clamped position (FIGS. 8 and 11). Alternatively, the automatic retraction of the firing member 1570 and/or the actuation of the retraction actuator can retract the firing member 1570 back into is proximal unactuated position to automatically open the end effector 1300 without requiring the clinician to release the closure actuator 1130. To move the second jaw 1320 into an open position (FIG. 12A), the proximal end 1321 of the second jaw 1320 comprises a camming surface 1339 defined on the proximal end 1321 and the firing member 1570 comprises a cam portion 1579 defined on a proximal end of the firing member 1570. The cam portion 1579 is configured to pivot the second jaw 1320 into the open position upon contacting the camming surface 1339.

Once the staple cartridge 1500 has been at least partially expended, i.e., at least partially fired, and the end effector 1500 has been re-opened, the staple cartridge 1500 can be removed from the first jaw 1310 and replaced with another staple cartridge 1500, and/or any other suitable staple cartridge. If the expended staple cartridge 1500 is not replaced, the firing drive 1600 is locked out from performing another staple firing stroke. Such a lockout can comprise an electronic lockout that prevents the control system from operating the drive motor 1610 to perform another staple firing stroke until the spent staple cartridge 1500 is replaced with an unspent staple cartridge. In addition to or in lieu of an electronic lockout, the surgical instrument 1000 can include a mechanical lockout which blocks the distal advancement of the firing member 1570 through another staple firing stroke unless the spent staple cartridge 1500 is replaced. Notably, referring to FIG. 12, the sled 1550 is not retracted proximally with the firing member 1570 after the staple firing stroke. As such, the electronic and/or mechanical lockout can key off of the position of the sled 1550 at the outset of the staple firing stroke. Stated another way, the staple firing stroke is prevented or blocked if the sled 1550 is not in its unfired position when the staple firing stroke is initiated.

Alternatively, a surgical instrument can comprise separate and distinct closure and staple firing drive systems. In some cases, the closure drive system comprises a closure actuator and a closure drive motor which, when actuated, moves the closure actuator distally through a closure stroke to close the end effector. In such cases, the staple firing drive system comprises a firing actuator and a separate firing drive motor which moves the firing actuator distally through a staple firing stroke. As discussed in greater detail below, the length of the closure stroke in such cases can be adjusted independently of the staple firing stroke to control the position of the second jaw 1320. Moreover, the closure drive system can also be actuated during the staple firing stroke to further control the position of the second jaw 1320.

As discussed above, the staples ejected from the staple cartridge 1500 can seal the incised patient tissue. That said, a single row of staples on each side of the incision may not be able to create a sufficient hemostatic seal in the incised patient tissue. To this end, as discussed in greater detail below, the surgical instrument 1000 is further configured to use electrical energy to seal the patient tissue. Referring to FIG. 1, the surgical instrument 1000 further comprises an energy delivery system 1900 including an off-board power supply and a cord 1990 in communication with the off-board power supply. Alternatively, the energy delivery system 1900 comprises an on-board power supply, such as the battery 1180, for example. In either event, the control system of the surgical instrument 1000 is configured to control the delivery of energy from the energy delivery system 1900 to the patient tissue, as discussed in greater detail below. The energy delivery system 1900 comprises an electrical circuit extending through the shaft 1200 and the articulation joint 1400 and into the end effector 1300. Referring to FIG. 6, the energy delivery system 1900 comprises an electrode supply circuit 1920 that extends into the second jaw 1320 and comprises a longitudinal electrode 1925 mounted to the frame 1325 of the second jaw 1320. The longitudinal electrode 1925 is electrically insulated from the metal frame 1325 such that the current flowing through the longitudinal electrode 1925, or at least a majority of the current flowing through the longitudinal electrode 1925, flows into a longitudinal return electrode 1590 of the staple cartridge 1500. The longitudinal return electrode 1590 is seated in the cartridge body 1510 and comprises a cartridge connector 1595 that engages, and makes an electrical connection with, a circuit connector 1915 of an electrode return circuit 1910 when the staple cartridge 1500 is seated in the first jaw 1310. Alternatively, the staple cartridge 1500 includes a supply electrode and the second jaw 1320 includes a return electrode.

Referring to FIG. 1, the surgical instrument 1000 further comprises a display 1190 in communication with the control system of the surgical instrument 1000. In various instances, the control system is configured to display parameters and/or data regarding the staple firing system and/or the energy delivery system.

FIGS. 14-16 depict a surgical instrument shaft assembly 25400 configured for use with a surgical instrument such as those disclosed herein, for example. The shaft assembly 25400 comprises many of the same components as the surgical instrument 1000. The shaft assembly 25400 may comprise various drive members configured to articulate an end effector, rotate an end effector about a longitudinal axis, and/or fire an end effector, for example. One or more of these drive members and/or components within a shaft assembly may be subject to tension and/or compression owing to the interaction of such drive members and/or components with other drive members and/or components of a surgical instrument employing the shaft assembly 25400. In at least one instance, articulation of an end effector may cause a spine member to which an articulation joint may be attached to stretch and/or compress upon articulation of the end effector. This can be attributed to the attachment of the articulation joint to the spine member and the bending, or articulation, of the articulation joint. A core insert may aid in strengthening the shaft assembly 25400 and/or help define a maximum system stretch of the shaft assembly 25400. The maximum system stretch may be defined by a maximum load and/or a maximum stretch length, for example. A core insert may prevent a member of a shaft assembly from prematurely failing. A core insert may also predefine the maximum system stretch of a shaft assembly so as to provide a predictable amount of stretch of one or more components of the shaft assembly and/or surgical instrument with which the shaft assembly is used.

The shaft assembly 25400 comprises a spine member 25410 and the articulation joint 1400. The shaft assembly 25400 further comprises a proximally extending articulation joint portion 25430 comprising pin apertures 25431. The spine member 25410 comprises lateral slots 25411 defined therein each configured to receive an articulation actuator. The lateral slots 25411 can provide space between an outer shaft tube and the spine member 25410 for the articulation actuators. The spine member 25410 further comprises a primary slot 25412 configured to receive a drive member therethrough such as, for example, a primary drive shaft.

The shaft assembly 25400 further comprises a core insert 25420 positioned with the spine member 25410. The core insert 25420 may be insert molded and/or overmolded into the spine member 25410. Other suitable manufacturing techniques are contemplated. The core insert 25420 comprises a proximal core member 25421 comprising a distal hook end 25422. The distal hook end 25422 comprises a hook tab 25423 extending from the proximal core member 25421. The core insert 25420 further comprises a distal core member 25425 comprising a proximal hook end 25426. The proximal hook end 25426 comprises a hook tab 25427 extending from the distal core member 25425. The hook tabs 25423, 25427 face each other and cooperate to transmit stretching forces to each other through the spine member 25410. The distal core member 25425 further comprises a distal mounting portion 25428 extending distally out of the spine member 25410. The distal mounting portion 25428 comprises pin apertures 25429 defined therethrough. The shaft assembly 25400 further comprises pins 2543 configured to pin the articulation joint portion 25430 to the distal mounting portion 25428 by way of apertures 25429. The pinned engagement between the distal mounting portion 25428 and the articulation joint portion 25430 may result in stretching, or tensile, forces being applied to the spine member 25410. The core insert 25420 may help prevent the spine member 25410 from overstretching, for example.

In at least one instance, the spine member 25410 comprises a first material and the core insert 25420 comprises a second material which is different than the first material. The first material may comprise a polymer material and the second material may comprise a metallic material. In at least one instance, the tensile strength of the second material is greater than the tensile strength of the first material. Such an arrangement can reduce weight, for example, of a surgical instrument which employs the shaft assembly 25400 while maintaining a desired system and/or shaft strength of the surgical instrument where significant actuation forces are present. For example, as an articulation actuator articulates the end effector and bends the articulation joint 1400, stretching forces are applied to the spine member 25410 and the core insert 25420 can serve to counter these stretching forces. The material of the spine member 25410 positioned between the proximal core member 25421 and the distal core member 25425 may reduce capacitive coupling and/or electrically isolate the proximal core member 25421 from the distal core member 25425.

Although various devices have been described herein, modifications and variations may be implemented. Particular features, structures, or characteristics may be combined in any suitable manner. Where materials are disclosed for certain components, other materials may be used. Furthermore, a single component may be replaced by multiple components, and multiple components may be replaced by a single component, to perform a given function or functions.

The devices disclosed herein can be designed to be disposed of after a single use, or they can be designed to be used multiple times. In either case, however, a device can be reconditioned for reuse after at least one use. Reconditioning can include any combination of the steps including, but not limited to, the disassembly of the device, followed by cleaning or replacement of particular pieces of the device, and subsequent reassembly of the device. In particular, a reconditioning facility and/or surgical team can disassemble a device and, after cleaning and/or replacing particular parts of the device, the device can be reassembled for subsequent use. Those skilled in the art will appreciate that reconditioning of a device can utilize a variety of techniques for disassembly, cleaning/replacement, and reassembly. Use of such techniques, and the resulting reconditioned device, are all within the scope of the present application.

The devices disclosed herein may be processed before surgery. First, a new or used instrument may be obtained and, when necessary, cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, and/or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta radiation, gamma radiation, ethylene oxide, plasma peroxide, and/or steam.

## Claims

1. A surgical instrument assembly (1000), comprising:
a shaft (25400);
an articulation region (1400); and
an end effector (1500) attached to the shaft (25400) by way of the articulation region (1400), wherein the end effector (1500) is configured to be articulated relative to the shaft (25400), wherein the shaft (25400) comprises:
an outer housing (1210);
an inner spine (25410) positioned with the outer housing (1210); and
a core member (25420) positioned within the inner spine (25410), wherein the core member (25420) comprises:
a distal end attached to a component of the end effector (1500); and
a proximal end attached to a proximal housing of the surgical instrument assembly (1000), wherein the core member (25420) comprises a material different than a material of the inner spine (25410), and wherein the core member (25420) defines a maximum system stretch of the inner spine (25410);
**characterized in that** the core member (25420) comprises a proximal core member (25421) and a distal core member (25425), wherein the proximal core member (25421) is attached to the proximal housing and the distal core member (25425) is attached to the component of the end effector (1500).

2. The surgical instrument assembly (1000) of Claim 1, wherein the proximal core member (25421) comprises a distal hook portion (25422) and the distal core member (25425) comprises a proximal hook portion (25426), wherein the proximal core member and the distal core member comprise a material different than a material of the inner spine.

3. The surgical instrument assembly (1000) of Claim 2, wherein the distal hook portion (25422) and the proximal hook portion (25426) are configured to interact with each other to transfer stretching forces therebetween.

4. The surgical instrument assembly (1000) of Claim 3, wherein the inner spine (25410) is configured to isolate the proximal hook portion (25426) from the distal hook portion (25422).

5. The surgical instrument assembly (1000) of any one of Claims 2-4, wherein the distal hook portion (25422) is a distal end of the proximal core member (25421) and the proximal hook portion (25426) is a proximal end of the distal core member (25425).

6. The surgical instrument assembly (1000) of any preceding Claim, wherein the inner spine (25410) comprises a slot (25411) defined therein configured to receive an articulation actuation member.

7. The surgical instrument assembly (1000) of any preceding Claim, wherein the inner spine (25410) comprises a drive channel (25412) defined therein configured to receive a drive member.

8. The surgical instrument assembly (1000) of any preceding Claim, wherein the inner spine (25410) comprises an electrically insulative material.

9. The surgical instrument assembly (1000) of any preceding Claim, wherein the core member (25420) comprises a metallic material.

10. The surgical instrument assembly (1000) of Claim 9, wherein the proximal core member (25421) and the distal core member (25425) comprise a metallic material.

11. The surgical instrument assembly (1000) of Claim 9, wherein the core member (25420) has a greater tensile strength than the inner spine (25410).

12. The surgical instrument assembly (1000) of Claim 10, wherein the proximal core member (25421) and the distal core member (25425) have a greater tensile strength than the inner spine (25410).

13. The surgical instrument assembly (1000) of any one of Claims 1 - 8, wherein the proximal core member (25421) and the distal core member (25425) are insert molded within the inner spine (25410).

## Patentansprüche

1. Chirurgische Instrumentenanordnung (1000), umfassend:
einen Schaft (25400),
einen Gelenkbereich (1400), und
einen Endeffektor (1500), der über den Gelenkbereich (1400) an dem Schaft (25400) angebracht ist, wobei der Endeffektor (1500) dazu ausgelegt ist, bezogen auf den Schaft (25400) ausgelenkt zu werden, wobei der Schaft (25400) umfasst:
ein Außengehäuse (1210),
einen Innenrücken (25410), der in dem Außengehäuse (1210) positioniert ist; und
ein Kernelement (25420), das in dem Innenrücken (25410) positioniert ist, wobei das Kernelement (25420) umfasst:
ein distales Ende, das an einer Komponente des Endeffektors (1500) angebracht ist, und
ein proximales Ende, das an einem proximalen Gehäuse der chirurgischen Instrumentenanordnung (1000) angebracht ist, wobei das Kernelement (25420) ein Material umfasst, das sich von einem Material des Innenrückens (25410) unterscheidet, und wobei das Kernelement (25420) eine maximale Systemdehnung des Innenrückens (25410) definiert,
**dadurch gekennzeichnet, dass** das Kernelement (25420) ein proximales Kernelement (25421) und ein distales Kernelement (25425) umfasst, wobei das proximale Kernelement (25421) an dem proximalen Gehäuse angebracht ist und das distale Kernelement (25425) an der Komponente des Endeffektors (1500) angebracht ist.

2. Chirurgische Instrumentenanordnung (1000) nach Anspruch 1, wobei das proximale Kernelement (25421) einen distalen Hakenabschnitt (25422) umfasst und das distale Kernelement (25425) einen proximalen Hakenabschnitt (25426) umfasst, wobei das proximale Kernelement und das distale Kernelement ein Material umfassen, das sich von dem Material des Innenrückens unterscheidet.

3. Chirurgische Instrumentenanordnung (1000) nach Anspruch 2, wobei der distale Hakenabschnitt (25422) und der proximale Hakenabschnitt (25426) dazu ausgelegt sind, miteinander zu interagieren, um Dehnungskräfte zwischen ihnen zu übertragen.

4. Chirurgische Instrumentenanordnung (1000) nach Anspruch 3, wobei der Innenrücken (25410) dazu ausgelegt ist, den proximalen Hakenabschnitt (25426) von dem distalen Hakenabschnitt (25422) zu trennen.

5. Chirurgische Instrumentenanordnung (1000) nach einem der Ansprüche 2-4, wobei der distale Hakenabschnitt (25422) ein distales Ende des proximalen Kernelements (25421) ist und der proximale Hakenabschnitt (25426) ein proximales Ende des distalen Kernelements (25425) ist.

6. Chirurgische Instrumentenanordnung (1000) nach einem vorhergehenden Anspruch, wobei der Innenrücken (25410) einen darin definierten Schlitz (25411) umfasst, der dazu ausgelegt ist, ein Gelenkbetätigungselement aufzunehmen.

7. Chirurgische Instrumentenanordnung (1000) nach einem vorhergehenden Anspruch, wobei der Innenrücken (25410) einen darin definierten Antriebskanal (25412) umfasst, der dazu ausgelegt ist, ein Antriebselement aufzunehmen.

8. Chirurgische Instrumentenanordnung (1000) nach einem vorhergehenden Anspruch, wobei der Innenrücken (25410) ein elektrisch isolierendes Material umfasst.

9. Chirurgische Instrumentenanordnung (1000) nach einem vorhergehenden Anspruch, wobei das Kernelement (25420) ein metallisches Material umfasst.

10. Chirurgische Instrumentenanordnung (1000) nach Anspruch 9, wobei das proximale Kernelement (25421) und das distale Kernelement (25425) ein metallisches Material umfassen.

11. Chirurgische Instrumentenanordnung (1000) nach Anspruch 9, wobei das Kernelement (25420) eine größere Dehnfestigkeit hat als der Innenrücken (25410).

12. Chirurgische Instrumentenanordnung (1000) nach Anspruch 10, wobei das proximale Kernelement (25421) und das distale Kernelement (25425) eine größere Dehnfestigkeit haben als der Innenrücken (25410).

13. Chirurgische Instrumentenanordnung (1000) nach einem der Ansprüche 1-8, wobei das proximale Kernelement (25421) und das distale Kernelement (25425) in den Innenrücken (25410) eingegossen sind.

## Revendications

1. Ensemble instrument chirurgical (1000), comprenant :
un arbre (25400) ;
une région d'articulation (1400) ; et
un effecteur terminal (1500) fixé à l'arbre (25400) par l'intermédiaire de la région d'articulation (1400), l'effecteur terminal (1500) étant configuré pour être articulé par rapport à l'arbre (25400), l'arbre (25400) comprenant :
un boîtier extérieur (1210) ;
une colonne interne (25410) positionnée avec le boîtier extérieur (1210) ; et
un élément central (25420) positionné dans la colonne interne (25410), l'élément central (25420) comprenant :
une extrémité distale fixée à un composant de l'effecteur terminal (1500) ; et
une extrémité proximale fixée à un boîtier proximal de l'ensemble instrument chirurgical (1000), l'élément central (25420) comprenant un matériau différent de celui de la colonne interne (25410), et l'élément central (25420) définissant un étirement maximal de la colonne interne (25410) ;
**caractérisé en ce que** l'élément central (25420) comprend un élément central proximal (25421) et un élément central distal (25425), l'élément central proximal (25421) étant fixé au boîtier proximal et l'élément central distal (25425) étant fixé au composant de l'effecteur terminal (1500).

2. Ensemble instrument chirurgical (1000) selon la revendication 1, l'élément central proximal (25421) comprenant une partie de crochet distale (25422) et l'élément central distal (25425) comprenant une partie de crochet proximale (25426), l'élément central proximal et l'élément central distal comprenant un matériau différent d'un matériau de la colonne interne.

3. Ensemble instrument chirurgical (1000) selon la revendication 2, la partie de crochet distale (25422) et la partie de crochet proximale (25426) étant configurées pour interagir l'une avec l'autre afin de transférer les forces d'étirement entre elles.

4. Ensemble instrument chirurgical (1000) selon la revendication 3, la colonne interne (25410) étant configurée pour isoler la partie de crochet proximale (25426) de la partie de crochet distale (25422).

5. Ensemble instrument chirurgical (1000) selon l'une quelconque des revendications 2 à 4, la partie de crochet distale (25422) étant une extrémité distale de l'élément central proximal (25421) et la partie de crochet proximale (25426) étant une extrémité proximale de l'élément central distal (25425).

6. Ensemble instrument chirurgical (1000) selon n'importe quelle revendication précédente, la colonne interne (25410) comprenant une fente (25411) définie à l'intérieur configurée pour recevoir un élément d'actionnement d'articulation.

7. Ensemble instrument chirurgical (1000) selon n'importe quelle revendication précédente, la colonne interne (25410) comprenant un canal d'entraînement (25412) défini à l'intérieur, configuré pour recevoir un élément d'entraînement.

8. Ensemble instrument chirurgical (1000) selon n'importe quelle revendication précédente, la colonne interne (25410) comprenant un matériau électriquement isolant.

9. Ensemble instrument chirurgical (1000) selon n'importe quelle revendication précédente, l'élément central (25420) comprenant un matériau métallique.

10. Ensemble instrument chirurgical (1000) selon la revendication 9, l'élément central proximal (25421) et l'élément central distal (25425) comprenant un matériau métallique.

11. Ensemble instrument chirurgical (1000) selon la revendication 9, l'élément central (25420) ayant une résistance à la traction supérieure à celle de la colonne interne (25410).

12. Ensemble instrument chirurgical (1000) selon la revendication 10, l'élément central proximal (25421) et l'élément central distal (25425) ayant une résistance à la traction supérieure à celle de la colonne interne (25410).

13. Ensemble instrument chirurgical (1000) selon l'une quelconque des revendications 1 à 8, l'élément central proximal (25421) et l'élément central distal (25425) étant un insert moulé dans la colonne interne (25410).
